Europäisches Patentamt

European Patent Office

Office européen des brevets

(19)

(11) Veröffentlichungsnummer: **0 250 994 B1**

(12) # EUROPÄISCHE PATENTSCHRIFT

(45) Veröffentlichungstag der Patentschrift: **22.07.92**

(51) Int. Cl.⁵: **A61K 47/14**

(21) Anmeldenummer: **87108532.0**

(22) Anmeldetag: **12.06.87**

(54) **Neue Mittel zur Abdeckung unverletzter und/oder verletzter Bereiche menschlicher oder tierischer Haut.**

(30) Priorität: **20.06.86 DE 3620685**

(43) Veröffentlichungstag der Anmeldung:
**07.01.88 Patentblatt 88/01**

(45) Bekanntmachung des Hinweises auf die
Patenterteilung:
**22.07.92 Patentblatt 92/30**

(84) Benannte Vertragsstaaten:
**AT BE CH DE ES FR GB IT LI NL SE**

(56) Entgegenhaltungen:
**EP-A- 0 100 981**
**DE-A- 2 418 362**
**FR-A- 3 000**
**FR-A- 2 126 270**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Bartnik, Friedhelm, Dr.**
**Melanchthonstr. 11**
**W-4000 Düsseldorf(DE)**
Erfinder: **Hachmann, Klaus, Dr.**
**Am Eichelkamp 14**
**W-4010 Hilden(DE)**
Erfinder: **Lintner, Karl, Dr.**
**Kühlwetterstr. 10**
**W-4000 Düsseldorf(DE)**
Erfinder: **Pittermann, Wolfgang, Dr.**
**Schwarzbachstr. 33**
**W-4000 Düsseldorf(DE)**
Erfinder: **Ritter, Wolfgang, Dr.**
**Am Bandenfeld 74**
**W-5657 Haan(DE)**

**Beschreibung**

Die Erfindung betrifft neue fließfähige bis feste Mittel zur Abdeckung von unverletzten und/oder verletzten Hautpartien auf Basis eines körperverträglichen Trägermaterials. Die Erfindung ist insbesondere gekennzeichnet durch die Verwendung bestimmt ausgewählter oligomere Kondensationsprodukte niederer Hydroxycarbonsäuren, die dieses Trägermaterial verkörpern und dabei in der Anwendung der erfindungsgemäß zusammengestellten Zubereitungen die verschiedenartigsten Funktionen übernehmen können. Die Oligomerprodukte können nicht nur die Konsistenz der Mittel bestimmen, ihre Mitverwendung und Anpassung an den jeweiligen Anwendungszweck ermöglicht die Herstellung von dünnflüssigen, über salbenartig pastöse, wachsartige und schließlich bis hin zu festen Zubereitungen. Die oligomeren Trägermaterialien können dabei in der Anwendung auf der Haut als geschlossener Filmbildner oder auch in der Form von Pulvern bzw. luftigen Pudern erscheinen. Übereinstimmend zeichnen sich alle diese Materialien durch ihre hohe Körperverträglichkeit und durch ihre Körperresorbierbarkeit aus. Die erfindungsgemäß eingesetzten Oligomertypen werden über Stoffwechselvorgänge des Körpers abgebaut und verwertet. Die Erfindung ermöglicht dabei aber insbesondere eine gezielte Einflußnahme über die Permanenz der auf die Hautpartien aufgebrachten oligomeren Trägerstoffe, d. h. über den Zeitraum der Wahrnehmbarkeit dieser Materialien an der Hautoberfläche. Es gelingt insbesondere in Abstimmung mit der Resorptionsfähigkeit der jeweils betroffenen Hautpartien gezielt beispielsweise salbenartig pastöse Zubereitungen herzustellen, die als dünner Aufstrich auf eine Hautpartie aufgebracht, für einen vorbestimmten Zeitraum - beispielsweise 10 - 15 Stunden - auf der Hautoberfläche verbleiben, bzw. wahrnehmbar sind, bevor sie in das Innere des Hautgefüges weggeschlagen sind. Die neuen oligomeren Komponenten eignen sich damit ganz allgemein als Trägermaterialien für die Anwendung von Wirkstoffen auf der Haut, sei es z.B. im Bereich der Kosmetik, der Pharmakologie oder der Desinfektion. Die Oligomermaterialien können dabei zur Fixierung von Wirkstoffen für vorbestimmte Zeiträume auf Hautpartien eingesetzt werden, sie können aber selber auch wichtige Funktionen beispielsweise in der Wundversorgung oder in der kosmetischen Versorgung unverletzter Hautpartien übernehmen.

Es ist bekannt, daß hochpolymere Ester ausgewählter niederer Hydroxycarbonsäuren und zwar insbesondere der Milchsäure hohe Körperverträglichkeit besitzen und in der Operationstechnik beispielsweise als körperverträgliches und körperresorbierbares Fadenmaterial eingesetzt werden. Im Laufe von Wochen bzw. Monaten werden diese hochpolymeren Lactide abgebaut und schließlich über den Körperstoffwechsel aus dem Körper ausgeschwemmt. Auch die Implantation vorgeformter Feststoffteile aus Polymilchsäure hohen Molekulargewichts in den menschlichen und/oder tierischen Körper ist intensiv untersucht worden und der sich meist über Monate erstreckende Abbau dieser Implantate im Körpergeschehen erfolgt ohne nachhaltige Störung des betroffenen Organismus.

Die Lehre der vorliegenden Erfindung geht von der Überlegung aus, die pharmakologische Unbedenklichkeit solcher ausgewählter Polyester und die Fähigkeit des lebenden Organismus zur Resorption durch körpereigene Abbauvorgänge an dieser Substanzklasse in einem Bereich des Molekulargewichts der hier betroffenen Ester auszunutzen, der weit unter dem bisher hierfür benutzter Materialien liegt. Die Erfindung will insbesondere körperresorbierbare Produkte mit einem Konsistenzspektrum von dünnflüssig über pastös, wachsartig bis hin zu Feststoffen einsetzen und dabei diese körperverträglichen und körperresorbierbaren Oligoester in vielfältiger Weise als Trägermaterial - das aber durchaus aber auch eine eigene Funktion in der Anwendung besitzen kann - verwerten.

Gegenstand der Erfindung ist dementsprechend in einer ersten Ausführungsform die Verwendung von fließfähigen bis festen oligomeren Estern der Milchsäure und/oder der Glykolsäure und/oder ihren dermatologisch verträglichen Derivaten als resorbierbarer Träger und/oder Filmbildner in Mitteln zur Abdeckung unverletzter und/oder verletzter Bereiche menschlich oder tierischer Haut. Die erfindungsgemäßen Oligomeren zeichnen sich dabei durch einen mittleren Oligomerisationsgrad der gewählten Oxycarbonsäuren bis zu etwa 100 und vorzugsweise bis zu etwa 50 aus, wobei ein mittlerer Oligomerisationsgrad bis zu etwa 35 Einheiten der gewählten Hydroxycarbonsäure unter Berücksichtigung der im folgenden geschilderten weiteren Kombinationsmöglichkeiten der Oligomeren mit körperverträglichen anderen Komponenten bereits ein befriedigendes Konsistenzspektrum von dünnflüssig bis hin zu fest ermöglicht. Wie die nachfolgenden Beispiele zeigen, sind in charakteristischen Oligomerderivaten Oligomerisationsgrade schon bis zu 25 und insbesondere im Bereich von etwa 2 bzw. etwa 3 bis 25 geeignet, einerseits dünnflüssige Öle, ebenso aber salbenartig pastöse bzw. cremeartige Massen, andererseits bis hin zu festen harten, zu Pulvern aufarbeitbaren Stoffen zu ermöglichen.

Glykolsäure und Milchsäure sind bekanntlich niedere Hydroxycarbonsäuren, die im gesunden Stoffwechselgeschehen des lebenden Organismus auftreten und vom Körper verarbeitet bzw. ausgeschieden werden können. Die Milchsäure kann dabei in Form ihres Racemats oder auch in Form ihrer optischen

2

Antipoden - hier insbesondere in Form der im natürlichen Stoffwechselgeschehen auftretenden 1-Form - oder als beliebige Gemische der optischen Antipoden Verwendung finden.

Oligomere bzw. Polymere der Hydroxycarbonsäuren weisen an sich auf Grund der Monomerstruktur ebenso wie die Monomeren einen Restgehalt an freien Hydroxylgruppen und freien Carboxylgruppen auf. Für die Erfindung kann insbesondere das Vorhandensein freier Carboxylgruppen eine beträchtliche Rolle spielen. Vorliegende freie Carboxylgruppen führen bei der Anwendung erfindungsgemäßer Behandlungsmittel auf Wundbereiche, insbesondere noch blutende frische Wunden zu einer Blut-Koagulation und damit gegebenenfalls zu einer erwünschten Wundversorgung. Das Vorliegen freier Carboxylgruppen kann andererseits auch den pH-Wert der Massen in den dermatologisch häufig erwünschten schwach sauren Bereich verschieben, sodaß auch bei der Anwendung der neuen Mittel auf der unverletzten Haut mittels freier Carboxylgruppen durchaus erwünschte Effekte ausgelöst werden können. Auf der anderen Seite wird aber in zahlreichen Anwendungsfällen bewußt auf das Vorliegen insbesondere freier Carboxylgruppen verzichtet werden.

Für diesen Fall und ganz allgemein für die Derivatisierung der Oligomeren der erfindungsgemäßen Art bieten sich die beiden Ester bildenden Gruppen der Monomeren bzw. der Oligomeren an, d. h. die Hydroxylgruppe einerseits und die Carboxylgruppe andererseits. Beide reaktiven Stellen sind zur Ausbildung einfacher chemischer Abwandlungen geeignet, wobei hier wieder solchen Derivaten besondere Bedeutung zukommt, die die erwünschte Körperverträglichkeit und/oder Resorbierbarkeit der Materialien durch den Stoffwechsel nicht beeinträchtigen. Im Gegenteil kann durch die Mitverwendung körperverträglicher Reaktanten wichtiger Einfluß auf die eingangs geschilderte Möglichkeit der Steuerung der Permanenz des Materials auf der Hautoberfläche genommen werden. Für die Derivatisierung der Oligomeren bietet sich insbesondere die Veresterung mit hydroxylgruppenhaltigen bzw. carboxylgruppenhaltigen Reaktanten an, die die geschilderten Voraussetzungen insbesondere die Körperverträglichkeit erfüllen.

Die oligomeren Milchsäure- und/oder Glykolsäureester können dementsprechend mit ein- und/oder mehrfunktionellen Alkoholen und/oder mit ein- und/oder mehrfunktionellen Carbonsäuren verknüpft sein. Im Falle der alkoholischen Verknüpfung eignen sich in bevorzugten Ausführungsformen entsprechende Ester mit Alkoholen mit bis zu 4, insbesondere mit bis zu 3 Hydroxylgruppen, wobei hier einerseits einwertige Alkohole besondere Bedeutung besitzen können, andererseits zwei- und insbesondere dreiwertigen Alkoholen eine Schlüsselstellung zukommen kann. Im zuletzt genannten Fall sind es insbesondere Glyceride,die unter Mitverwendung der erfindungsgemäßen Glykolsäure- und/oder Milchsäureoligomeren zu außerordentlich vielgestaltigen und hochinteressanten Produkten bzw. Produkteigenschaften führen. Auf dem Gebiet der Mitverwendung von Carbonsäuren können einerseits physiologisch verträgliche Carbonsäuren, hier insbesondere Monocarbonsäuren interessant sein, aber auch polyfunktionelle Carbonsäuren, beispielsweise Di- oder Tricarbonsäuren führen zu interessanten und physiologisch unbedenklichen Oligomerderivaten für vielgestaltige Anwendungszwecke.

Die Herstellung der oligomeren Hydroxycarbonsäuren bzw. ihrer Derivate erfolgt in an sich bekannter Weise. Oligomere der Glykolsäure und/oder Milchsäure ohne Mitverwendung von weiteren Reaktanten können beispielsweise durch bekannte Kondensation der monomeren und/oder dimeren Ausgangsmaterialien - beispielsweise des aus zwei Milchsäureresten gebildeten Lactids - hergestellt werden. Die Polykondensationsreaktion wird dabei üblicherweise durch Erhitzen des Einsatzmaterials auf eine Temperatur oberhalb seines Schmelzpunktes in Gegenwart von Katalysatoren, beispielsweise in Gegenwart mehrwertiger Metalloxide oder ihrer Verbindungen unter wasserfreien Bedingungen in einer Inertgasatmosphäre durchgeführt. Geeignete Katalysatoren sind beispielsweise Zinnoxid oder Zinnsalze wie Zinncarbonat, basisches Zinncarbonat, Zinndiethyl, Aluminium-, Titan-, Magnesium-, Barium- und/oder Bleiverbindungen. Menge und Art des verwendeten Katalysators bestimmen die Verfahrenstemperatur und die Dauer der Umsetzung. Es kann zweckmäßig sein, das Reaktionsgemisch während der Polymerisation zu rühren, damit eine möglichst homogene Reaktion bzw. Reaktionsmischung sichergestellt sind. Die Umsetzung kann mehrstufig durchgeführt werden derart, daß in mehrstufiger Temperaturführung und/oder unter mehrstufigen Druckbedingungen gearbeitet wird, wobei beispielsweise zu Anfang der Reaktion bei Normaldruck und im Verlauf der fortschreitenden Reaktion unter verringerten Drucken gearbeitet wird. Die Polykondensationstemperatur wird bis zum gewünschten mittleren Oligomerisationsgrad vorangetrieben. Das Reaktionsprodukt kann dann in üblicher Weise von mitverwendeten Katalysatoranteilen befreit werden, sofern diese Katalysatoren Bedenken bei der Anwendung der Oligomerprodukte auf der Haut veranlassen.

In wichtigen Ausführungsformen der Erfindung werden neben und/oder an Stelle freier Hydroxycarbonsäuren deren reaktionsfähige Derivate insbesondere deren Ester mit niederen Alkoholen eingesetzt. So kann sowohl die Glykolsäure wie die Milchsäure beispielsweise in Form ihrer Ester mit Monoalkoholen mit 1-3 C-Atomen insbesondere als Ethylester Verwendung finden. Auf dem Gebiet der monofunktionellen Alkohole als Co-Reaktanten kann andererseits aber besondere Bedeutung auch gerade längerkettigen

3

EP 0 250 994 B1

Monoalkoholen zukommen, die sich beispielsweise der Gruppe der sogenannten Fettalkohole zuordnen lassen, d.h. Alkohole der Kohlenstoffzahl 8 - 22 und insbesondere 10 - 18 sind. Sie können dabei natürlichen und/oder synthetischen Ursprungs sein. Für die Körperverträglichkeit dieser Co-Reaktanten gelten die allgemeinen Erkenntnisse zu diesen Verbindungen. Sie werden im allgemeinen problemlos in den Stoffwechsel des Körpers einbezogen. Gehärtete längerkettige Alkohole beispielsweise von der Art des Talgalkohols sind befähigt, bestimmte Eigenschaftsparameter bezüglich Konsistenz und/oder bezüglich Permanenz des oligomeren Reaktionsproduktes auf der Haut einzustellen bzw. auszulösen. Insbesondere längerkettige Alkohole können sowohl gesättigt wie auch ungesättigt sein und gegebenenfalls pharmakologisch unbedenkliche funktionelle Gruppen bzw. Substituenten aufweisen.

Dabei können solche monofunktionellen Alkohole als Starterkomponenten verwendet werden und mit den Hydroxycarbonsäuren als solchen oder mit ihren reaktionsfähigen Derivaten beispielsweise ihren Estern mit niederen Alkoholen zur Umsetzung gebracht werden.

Bei der Verwendung von polyfunktionellen Alkoholen kommen insbesondere niedere polyfunktionelle Alkohole als Co-Reaktanten in Betracht. Zu nennen sind hier beispielsweise Ethylenglykol, Glycerin und Trimethylolpropan. Sowohl dem Ethylenglykol als insbesondere auch dem Glycerin kommen besondere Bedeutung zu. Es leuchtet sofort ein, daß das Glycerin ein besonders geeigneter Co-Reaktant ist, der die Möglichkeit eröffnet, oligomersubstituierte Mono-, Di- und/oder Triglyceride mit den verschiedenartigsten Eigenschaften bezüglich Konsistenz und Permanenz auf der Haut zur Verfügung zu stellen. Die im folgenden an Hand solcher Glyceride erörterten Gesetzmäßigkeiten gelten sinngemäß für die Anwendung auf andere polyfunktionelle Alkohole, so daß aus Gründen der rationellen Erfindungsbeschreibung auf die getrennte Erörterung vergleichbarer Erfindungselemente verzichtet wird.

Fette bzw. Fettstoffe aus der Natur oder aus synthetischen Herstellungsverfahren sind in der Regel Triglyceride aus der Umsetzung von Glycerin und Monocarbonsäuren. Modifizierte Fettstoffe können dabei durch partielle Spaltung von Triglyceriden bzw. durch partielle Veresterung von Glycerin mit einem Unterschuß an Monocarbonsäuren im Sinne der Monoglyceride bzw. Diglyceride erhalten werden. Durch den Einbau oligomerer Bausteine der Glykolsäure und/oder der Milchsäure in Glyceridbindung und insbesondere durch Einbau der oligomeren Strukturelemente in Fette bzw.Partialfette natürlichen und/oder synthetischen Ursprungs gelingt die Herstellung erfindungsgemäß geeigneter Trägersubstanzen in einer Palette nahezu unbeschränkter Breite. Es können ölige Substanzen bis hin zu Hartfetten ausgebildet werden. Besonders interessant kann der Bereich noch streichbarer viskos salbenartiger Substanzen bis hin zu berührungstrockenen, beispielsweise mehr wachsartigen Produkten sein. Ausgangspunkt können dabei natürliche Fette und/oder Öle sein, die lediglich durch Einkondensation von Milchsäure und/oder Glykolsäure zu den oligomermodifizierten Trägersubstanzen der Erfindung umgewandelt werden. Hier liegen dann also komplexe Mischester vor, die neben dem polyfunktionellen Alkohol und den oligomeren Hydroxycarbonsäurestruktureinheitendie ursprünglichen Monocarbonsäuren des eingesetzten Triglycerids aufweisen. Über die Beschaffenheit der Carbonsäuren höherer Kohlenstoffzahl - wie sie beispielsweise in natürlichen Ölen, Fetten und/oder Wachsen vorkommen - kann wiederum Einfluß auf das Beschaffenheitsbild des Oligomereinheiten enthaltenden Kondensationsproduktes genommen werden,so daß sich hier in weitestem Umfang Möglichkeiten auftun, die Konsistenz durch Vorgabe der Struktur des komplexen Polyesterproduktes vorherzubestimmen. Gleichzeitig damit ist es aber auch möglich, die Verweildauer der Masse auf der Haut, d.h. ihre Permanenz, dem jeweils gewünschten Anwendungszweck anzupassen. Eine normale beispielsweise in der Kosmetik oder in der Dermatologie eingesetzte Fettcreme ist innerhalb eines vergleichsweise kurzen Zeitraumes von beispielsweise 15 - 30 Minuten von der Haut derart aufgenommen, daß äußerlich ein zusammenhängender Fettfilm nicht mehr feststellbar ist. Das kann erwünscht sein. Es gibt aber zahlreiche Anwendungszwecke, in denen es umgekehrt erwünscht wäre, eine vergleichsweise lang dauernde Permanenz des Fettfilmes sicherzustellen, gleichzeitig aber die Gewißheit zu haben, daß nach einer vorbestimmbaren Zeit von beispielsweise 10 - 20 Stunden das Substrat dann doch von der Haut aufgenommen ist. Als Beispiel sei hier nur die Fixierung von Wirkstoffen auf der Haut gennant, wobei im Rahmen der Kosmetik pflegende, regenerierende und/oder desinfizierende Wirkstoffe und im medizinischen Anwendungsfall desinfizierende und/oder wundversorgende Wirkstoffe im Vordergrund stehen, während in der pharmakologischen Anwendung der über einen längeren Zeitraum wirksame Auftrag pharmakologischer Wirkstoffe erwünscht sein kann. Die Erfindung eröffnet hier neue Möglichkeiten zum gezielten Einsatz beliebiger Wirkstoffe auf unverletzten und/oder verletzten Hautbereichen an Mensch und Tier.

Als besonders interessantes Einsatzgebiet neben den zuletzt geschilderten pastösen salbenartigen Massen sei ein anderes Beispiel herausgegriffen: Für die Wundversorgung im Bereich der Selbstmedikation, aber auch im ärztlichen bzw. klinischen Bereich kann Wundpudern ein hoher Stellenwert zukommen. Tatsächlich existieren zahlreiche Feststoffpuder, die aber oft nur beschränkt anwendungsfähig sind. Das ist immer dann der Fall, wenn das puderbildende Feststoffmaterial nicht körperresorbierbar ist,so daß Puder-

4

EP 0 250 994 B1

partikel den Heilungsprozeß stören bzw. im Laufe des Heilungsprozesses vom Körper wieder ausgestoßen werden müssen. Wundpuder unter Verwendung der erfindungsgemäßen Trägermateriaien erfüllen demgegenüber die Voraussetzung in idealer Weise,momentan den gewünschten Verschluß der Wunde bei gleichzeitiger Atmungsfähigkeit zu ermöglichen, die Aufsaugung des Exudates zu bewirken und den Granulationsprozeß zu beschleunigen. Hier kann insbesondere die Variante der Erfindung wirksam sein, in der im oligomeren Träger freie Carboxylgruppen mitverwendet werden. Beim Auftrag eines solchen Puders auf die offene Wunde bildet sich unter Mitwirkung der Wundflüssigkeit eine Art künstlichen Schorfs, der den natürlichen Granulations- und Wundheilungsprozeß nicht stört.

Für die Mitverwendung von Carbonsäuren als Co-Reaktanten gelten analoge Überlegungen, wie sie zu den mono- und/oder polyfunktionellen Alkoholen gemacht worden sind. Aus der Struktur der die Oligomeren bildenden Hydroxycarbonsäuren und ihrer prinzipiell gegebenen Möglichkeit sowohl über die Carboxylgruppe wie über ihre Hydroxylgruppe Esterbindungen auszubilden, ist das verständlich.

Geeignete Monocarbonsäuren liegen dementsprechend beispielsweise im Bereich bis etwa C22, vorzugsweise im Bereich bis etwa C18, wobei natürlichen und/oder synthetischen gesättigten oder olefinisch ungesättigten Carbonsäuren im Bereich von etwa C10 - C18 besondere Bedeutung zukommen kann. Grundsätzlich sind aber auch niedere Monocarbonsäuren geeignet, sofern nicht dermatologische und/oder andere pharmakologische Bedenken entgegenstehen.

Entsprechende Überlegungen gelten für das Gebiet der Mitverwendung von Polycarbonsäuren, insbesondere Di- und/oder Tricarbonsäuren, wobei hier insbesondere entsprechende niedere Carbonsäuren mit insgesamt bis zu 10,vorzugsweise mit bis zu 6 C-Atomen besondere Bedeutung zukommen kann.

Durch gemeinsame Verwendung von polyfunktionellen und monofunktionellen Reaktanten auf der Seite der Alkohole und/oder der Carbonsäuren gelingt der Aufbau vergleichsweise komplexer Kondensationsprodukte, die sich erfindungsgemäß aber stets durch die Gegenwart oligomerer Milchsäure-und/oder Glykolsäureeinheiten auszeichnen, wobei diesen Oligomereinheiten - im Zusammenwirken mit den anderen bestimmt ausgesuchten Co-Reaktanten - tragende Bedeutung zukommt.

Zur Einstellung insbesondere der Feststoffkonsistenz kann erfindungsgemäß von einem weiteren Prinzip Gebrauch gemacht werden: Die in den oligomeren Reaktionsprodukten gegebenenfalls vorliegenden freien Carboxylgruppen können in ihre Salze umgewandelt werden. Hierzu werden bekannte pharmakologisch unbedenkliche Basen verwendet.

Die Ausgestaltung der letztlich zum Einsatz kommenden Wirkstoffgemische umfaßt das breite Gebiet der Versorgung gesunder, kranker und/oder verletzter Haut bzw. der medikamentösen Einwirkung auf den Organismus durch die Haut an Mensch und Tier. Dementsprechend breit gestreut sind die Möglichkeiten der Abmischung und Zubereitung von fertigen Wirkstoffgemischen unter Mitverwendung der erfindungsgemäßen Oligomerkomponenten. Für das erfindungsgemäß betroffene Gebiet der Hautversorgung ist von besonderem Vorteil, daß die oligomeren Reaktionsprodukte durch Maskierung aller funktionellen Gruppen gewissermaßen zu Neutralkörpern umgewandelt sein können, so daß ihre Abmischung mit beliebigen Wirkstoff- oder sonstigen Hilfskomponenten keine Schwierigkeiten bereitet. Auf der anderen Seite liegt besonderer Vorteil darin, daß spätestens bei dem hydrolytischen Angriff an der oligomeren Strukturin situ beschränkte Mengen freier Carboxylgruppen gebildet werden, die den pH-Wert der Hautoberfläche auf den an sich gewünschten schwach sauren Bereich absenken können. Wie bereits angegeben besteht zusätzlich die Möglichkleit der Mitverwendung freier Carboxylgruppen von Anfang an und damit die Auslösung gezielter Effekte insbesondere einer blutstillenden Koagulation.

Die Verträglichkeit der erfindungsgemäßen Oligomeren mit üblichen hautpflegenden Komponenten oder Hilfsmitteln, die in vergleichbaren Präparaten eingesetzt werden, ist gut. So können übliche Lösungs- und/oder Emulgiermittel zur Herstellung von Flüssigpräparaten eingesetzt werden, die dann beispielsweise als Spray oder als Flüssiglotion zur Verwendung kommen. Emulgierte Wirkstoffkonzentrate unter Mitverwendung der erfindungsgemäßen filmbildenden Trägersubstanzen können zur Applikation mit Wasser verdünnt und dann aufgetragen werden. Die Abmischung mit öligen oder salbenförmig pastösen üblichen Hautbehandlungs- bzw. Schutzmitteln ist bei Einsatz angepaßter Oligomersubstrate problemlos möglich.Durch die Mitverwendung der erfindungsgemäßen Oligomersubstrate kann dann aber beispielsweise die Permanenz des eingesetzten Stoffgemisches auf der Haut gezielt variiert, insbesondere verlängert werden. Pulverförmige bzw. puderförmige Aufbereitungen erfindungsgemäßer Oligomerkomponenten können mit anderen in der Haut- bzw. Wundversorgung eingesetzten puderförmigen Komponenten abgemischt werden, um bestimmte gewünschte Effekte zu verstärken, beispielsweise um die saugende Wirkung der Pderauflage gegenüber Sekret oder die Filmbildungstendenz zum Verschluß gegen das Eindringen von Krankheitskeimen zu verstärken.

Aus dem großen Bereich der Anwendbarkeit erfindungsgemäßer Wirkstoffgemische seien hier lediglich beispielhaft genannt: Kosmetika mit ihrem breiten Bereich pflegender, regenerierender oder rein ornamentiver Zusatzstoffe. Das Gebiet präventiv-medizinischer Schutzstoffe beispielsweise im Bereich der Kinderpflege, insbesondere Salben und/oder Puder, der Schutzsalben etwa gegen verstärkte UV-Einstrahlung mit erfindungsgemäß verlängerter Permanenz auf der Haut. Das große Gebiet der Desinfektion von unverletzter und/oder verletzter Haut einschließlich der Schleimhaut sowie schließlich das Gebiet der Applikation pharmakologischer Wirkstoffe durch die Haut mit verzögerter Freigabe der Atktivstoffe.

Erfindungsgemäß wird es möglich, sehr spezifische Anforderungen zu erfüllen, die mit bisher bekannten Mitteln nicht vergleichbar befriedigt werden konnten. Als Beispiel sei hier das Gebiet der Kuh-Euter-Desinfektion genannt: Die Präventiv-Desinfektion des Kuh-Euters fordert die Fixierung von Desinfektionsmitteln an den Zitzen und insbesondere im Bereich des Strichkanals für den Zeitraum von etwa 10 - 12 Stunden. Bei zweimaligem Melken pro Tag soll dann aber zum Zeitpunkt des Melkvorganges kein merklicher Rückstand des Desinfektionsmittels und/oder der zusammen damit eingesetzten Hilfs- und Trägerstoffe vorliegen. Bis heute ist in der Praxis eine nachhaltige Desinfektion des Euter-Bereiches entweder nur für einen kurzen Zeitraum von beispielsweise 2 - 3 Stunden möglich oder aber es müssen solche Hilfs- und Trägerstoffe eingesetzt werden, deren Permanenz weit oberhalb des gewünschten Zeitraumes von 10 - 12 Stunden liegt. So ist der Versuch gemacht worden, durch die Ausbildung von Filmen mittels Polymerverbindungen den länger dauernden Schutz gegen das Eindringen von Krankheitskeimen sicherzustellen. Damit stellt sich dann aber beim nächsten Melkvorgang die Aufgabe, zunächst den Zitzenbereich sorgfältig zu reinigen, andernfalls gelangen Reste der Polymerverbindung und damit Desinfektionsmittel in die Milch und verunreinigen diese. Erfindungsgemäß wird es erstmalig möglich, durch Einstellung der Permanenz des Trägerstoffes auf den gewünschten Zeitraum von 10 - 12 Stunden die einfache und sichere Applikation üblicher Desinfektionsmittel in erfindungsgemäßen Trägern zu verwirklichen, wobei sich ein schützender Film ausbildet, der zum Zeitpunkt des nächsten Melkvorganges - aber eben auch erst dann vom Eutergewebe aufgenommen ist und damit beim nächsten Melkvorgang nicht mehr störend in Erscheinung tritt.

Die erfindungsgemäßen Mittel zur Abdeckung von unverletzten und/oder verletzten Hautpartien können das Oligomersegmente enthaltende Trägermaterial in Mengen von etwa 5 bis nahezu 100 % enthalten, wobei auch schon die Applikation in Abwesenheit sonstiger Hilfsstoffe - d.h. die Anwendung der reinen Oligomersubstanz - interessant sein kann. Die Menge der jeweils zugesetzten Wirkstoffe richtet sich nach den allgemeinen Forderungen des betroffenen Sachgebietes. Stark desinfizierende Wirkstoffe - beispielsweise keimtötende antibiotische und/oder sulfonamidhaltige Wirkstoffe - können schon in geringsten Konzentrationen von 0,1 - 1 Gew.-% hohe Wirkung entfalten, andere Desinfektionsmittel beispielsweise solche oxidierender oder nicht-oxidierender Art wie Wasserstoffperoxid, Harnstoffperhydrat, Benzoylperoxid, Glucoseoxidase, Milchsäure, PVP-Jod, Chlorhexidin, quartäre Ammoniumverbindungen, Undecylensäureamide, Lysozym, Oxoferin und dergleichen können gewünschtenfalls in größeren Mengen mitverwendet werden. Beispiele für pflegende und/oder heilende Wirkstoffe sind etwa Vitamin E, Vitamin C, Allantoin, Propolis, Pantothenylalkohol, Melissa officinalis, Oxoferin und dergleichen. In puderförmigen Zubereitungen können als zusätzlich sekretsaugende Komponenten Saccharin, d-Lactose aber auch anorganische Puderkomponenten wie Aerosil, Zinkoxid und dergleichen mitverwendet werden. Geeignete Lösungsmittel zur Herstellung von Flüssigzubereitungen sind insbesondere niedere Alkhohole, beispielsweise Ethanol, Isopropanol, n-Propanol, Propylenglykol, Glycerin und dergleichen.

Beispiele

Die nachfolgenden Beispiele schildern zunächst die Herstellung einer Mehrzahl von oligomeren Hydroxycarbonsäurederivaten mit der zugehörigen physikalischen Zustandsbeschreibung. Dabei sind diese Arbeiten unterteilt in die nachfolgenden 5 Stoffklassen:
1. Umsetzungsprodukte von Di- und Triolen mit Hydroxycarbonsäurederivaten
   a) Ethylenglykol/Glycerin x Glykolsäure
   b) Ethylenglykol/Glycerin x Milchsäure
   c) Ethylenglykol/Glycerin x Milchsäureethylester
   d) Ethylenglykol/Glycerin x Glykolsäure/Milchsäureethylester
   e) Ethylenglykol/Glycerin x Lactid
2. Umsetzungsprodukte von monofunktionellen Alkoholen mit Hydroxycarbonsäurederivaten
   a) Monoalkohol x Glykolsäure
   b) Monoalkohol x Milchsäure
   c) Monoalkohol x Milchsäureethylester
   d) Monoalkohol x Lactid
3. Umsetzungsprodukte von Dicarbonsäuren mit Hydroxycarbonsäuren

a) Adipinsäure x Glykolsäure

4. Umsetzungsprodukte von Glycerin mit Hydroxycarbonsäuren und Fettsäuren

a) Glycerin x Glykolsäure x Fettsäure

b) Glycerin x Milchsäure x Fettsäure

c) Glycerin x Glykolsäure/Milchsäure x Fettsäure

5. Umsetzungsprodukte von Mono- und Difettsäureglyceriden mit Lactid

a) Glycerinmonoester x Lactid

b) Glycerindiester x Lactid.

1. Umsetzungsprodukte von Di- und Triolen mit Hydroxycarbonsäurederivaten

a) Ethylenglykol/Glycerin x Glykolsäure

Herstellvorschrift:

In einem Dreihalskolben mit Rührer und Destillationsbrücke werden Glykolsäure und Ethylenglykol bzw. Glycerin vorgelegt. Unter Stickstoff wird schnell auf 150 °C und dann im Verlauf von 6 Stunden von 150 auf 200 °C hochgeheizt. Dabei spaltet sich bereits der größte Anteil des Reaktionswassers, der den Umsatz der Esterkondensation anzeigt, ab. Man läßt den Ansatz auf etwa 150 °C abkühlen, evakuiert vorsichtig auf 10 Torr und vervollständigt den Umsatz bei 200 °C und 10 Torr. Nach 30 Minuten wird das Produkt bei ca. 150 °C heiß abgefüllt. Die Zusammensetzung der Ansätze und die Oligomereigenschaften sind der Tabelle 1 zu entnehmen.

Tabelle 1

Unsetzungsprodukte aus Ethylenglykol / Glycerin und Glykolsäure

| Beispiel | Di-, Triol | Molverhältnis Glykolsäure/ Di- bzw. Triol | Molekulargewicht (theor.) | Viskosität | Farbe |
|---|---|---|---|---|---|
| 1 | Ethylenglykol | 3 : 1 | 236 | flüssig-viskos | farblos |
| 2 | " | 4 : 1 | 294 | viskos | gelblich |
| 3 | " | 6 : 1 | 410 | pastenartig | weiß |
| 4 | Glycerin | 3 : 1 | 266 | hochviskos | gelblich |
| 5 | " | 6 : 1 | 440 | hochviskos, pastenartig | gelblich |

1. b) Ethylenglykol/Glycerin x Milchsäure

Herstellvorschrift:

Die in der Tabelle 2 angegebenen Mengen Milchsäure und Ethylenglykol wurden in einer Laborrührapparatur mit Destillationsbrücke unter Stickstoff innerhalb 2 - 4 Stunden langsam auf 210 - 220 °C aufgeheizt. Dabei destillierte das Reaktionswasser und gleichzeitig das Wasser aus der Milchsäure (90

%ig in $H_2O$) über.

Um die Reaktion zu vervollständigen, wurde anschließend auf ca. 150 °C abgekühlt und bei dieser Temperatur 30 Minuten lang Wasserstrahlvakuum angelegt.

Zur Reinigung des Produktes kühlte man auf ca. 90 °C und versetzte mit Bleicherde Tonsil LFF 80 (5 % bezogen auf Milchsäure), ließ 30 Minuten rühren und saugte dann den Ansatz über eine Heißdampfnutsche mit Filterhilfsmittel ab. Es wurde heiß abgefüllt.

Als Katalysator wurde Fascat 2001 (0,5 % bezogen auf Milchsäure) eingesetzt.

1. c) Ethylenglykol/Glycerin x Milchsäureethylester

Herstellvorschrift:

In einer wie unter 1. a) beschriebenen Apparatur wurden der Di- bzw. Triol und Milchsäureethylester vorgelegt, viermal evakuiert, jeweils mit Stickstoff belüftet und anschließend unter schwachem $N_2$-Strom bis auf 160 °C Badtemperatur aufgeheizt. Bei ca. 60 °C Sumpftemperatur wurden pro Mol Milchsäure-

## Tabelle 2

Umsetzungsprodukte aus Ethylenglykol / Glycerin mit Milchsäure

| Bei-spiel | Di-, Triol | Molverhältnis Milchsäure/ Diol | Molekular-gewicht (theor.) | OH-Zahl gemessen | Viskosität | Farbe |
|---|---|---|---|---|---|---|
| 6 | Ethylen-glykol | 3 | 278 | 343 | leicht viskos | hellgelb, klar |
| 7 | " | 24 | 1793 | 80 | hochviskos | gelb, klar |

ethylester 340 mg einer 30 gew.-%igen Lösung von NaOCH$_3$ in Methanol hinzugegeben. Danach heizte man langsam weiter auf, bis bei ca. 190 °C Badtemperatur (ca. 150 °C Sumpftemperatur) erstmals Ethanol überdestillierte. Die Badtemperatur wurde allmählich bis auf 230 °C erhöht. Als kein Ethanol mehr überdestillierte, ließ man das Reaktionsgemisch abkühlen und evakuierte dann durch Wasserstrahlvakuum. Man heizte wider langsam bis 150 °C auf und destillierte so das restliche Ethanol im Wasserstrahlvakuum ab. Das Produkt wurde noch heiß abgefüllt.

1. d) Ethylenglykol bzw. Glycerin x Glykolsäure und Milchsäureethylester

Herstellvorschrift:

Siehe 1. a), 1. c).

## Tabelle 3

Umsetzungsprodukte aus Ethylenglykol / Glycerin mit Milchsäureethylester

| Beispiel | Di- bzw. Triol | Molverhältnis Milchsäure-ethylester/ Diol bzw. Triol | Molekular-gewicht (theor.) | Viskosität | Farbe |
|---|---|---|---|---|---|
| 8 | Ethylen-glykol | 2 : 1 | 206 | dünnflüssig | gelb |
| 9 | " | 4 : 1 | 351 | leicht viskos | dunkel |
| 10 | " | 6 : 1 | 495 | viskos | dunkel |
| 11 | " | 10 : 1 | 787 | viskos | dunkel |
| 12 | Glycerin | 6 : 1 | 525 | viskos | dunkel |
| 13 | Glycerin | 12 : 1 | 957 | hochviskos | dunkel |

EP 0 250 994 B1

Tabelle 4

Umsetzungsprodukte aus Ethylenglykol bzw. Glycerin mit Glykolsäure und gleichzeitig Milchsäureethylester

| Beispiel | Edukte a | Edukte b | Edukte c | Molverhältnis a:b:c | Molekular-gewicht (theor.) | Viskosität | Farbe |
|---|---|---|---|---|---|---|---|
| 14 | Glykol-säure | Milchsäure-ethylester | Ethylen-glykol | 5:1:1 | 424 | viskos | gelblich |
| 15 | " | " | " | 4:2:1 | 438 | hochviskos | leicht gelblich |
| 16 | " | " | " | 10:2:1 | 786 | pastenartig | gelblich |
| 17 | " | " | " | 8:4:1 | 815 | pastenartig teilkristallin | gelblich |
| 18 | " | " | Glyce-rin | 5:1:1 | 454 | hochviskos | gelb |

1. e) Ethylenglykol/Glycerin x Lactid

Herstellvorschrift:

Lactid und Ethylenglykol bzw. Glycerin wurden in einer üblichen Laborapparatur unter Stickstoff und unter Rühren innerhalb von einer Stunde auf 195 °C erwärmt. Man ließ dann 3 Stunden bei 195 °C reagieren und füllte heiß ab. Als Katalysator war eine Sn-II-Chlorid-Lösung in Ether zugegen (7 ml einer Lösung von 2,5 g SnCl$_2$ in 1000 ml Ether bei der Umsetzung von 3 Mol Lactid mit einem Mol Ethylenglykol).

Tabelle 5

Umsetzungsprodukte aus Ethylenglykol bzw. Glycerin mit Lactid

| Bei-spiel | Di- bzw. Triol | Molverhält-nis Lactid/ Di- bzw. Triol | Moleku-large-wicht (theor.) | OH-Zahl (gemes-sen) | Viskosi-tät nach Brook-field | Spin-del | Dreh-zahl (min$^{-1}$) | Wasser-löslich-keit | Fließ-fähig-keit |
|---|---|---|---|---|---|---|---|---|---|
| 19 | Ethylen-glykol | 1,5 | 278 | 386 | 0,88 mPa·s | 5 | 50 | + | + |
| 20 | " | 3,0 | 494 | 191 | 50 mPa·s | 7 | 10 | - | + |
| 21 | " | 6,0 | 926 | 89,3 | 8000 mPa·s | 7 | 0,5 | - | - |
| 22 | " | 12,0 | 1790 | 45,6 | >8000 mPa·s | 7 | 0,5 | - | - |
| 23 | Glycerin | 1,5 | 308 | 487 | 16,1 mPa·s | 5 | 10 | + | + |
| 24 | " | 3,0 | 524 | 275 | 720 mPa·s | 7 | 1 | - | - |
| 25 | " | 6,0 | 956 | 151 | 8000 mPa·s | 7 | 0,5 | - | - |

+ = gegeben          - = nicht gegeben

2. Umsetzungsprodukte von monofunktionellen Alkoholen mit Hydroxycarbonsäurederivaten

a) Monoalkohol x Glykolsäure

Herstellvorschrift:

In einem 500-ml-Dreihalskolben mit KPG-Rührer mit PTFE-Blatt, Thermometer, $N_2$-Einleitung und Destillationsbrücke mit Kühler, Vorlagekolben und Blasenzähler wurden alle Edukte vorgelegt. Das Reaktionsgefäß wurde viermal evakuiert, jeweils mit $N_2$ belüftet und anschließend unter schwachem Stickstoffstrom bis auf 180 °C Badtemperatur aufgeheizt. Bei ca. 80 °C Sumpftemperatur schmolz das Reaktionsgemisch auf. Danach wurde die Badtemperatur jeweils im Verlauf von 15 Min. um 10 °C erhöht, bis 230 °C erreicht wurden. Ab 190 °C Badtemperatur (155 °C Sumpftemperatur) destillierte Reaktionswasser ab. Zum Schluß der Reaktion wurden 200 °C Sumpftemperatur erreicht. Dann ließ man das Reaktionsgemisch bis auf 150 °C abkühlen und destillierte unter Wasserstrahlvakuum das restliche $H_2O$ ab. Das Produkt wurde noch heiß unter Stickstoff abgefüllt. Übliche Gesamtreaktionszeiten betrugen 3 bis 4 Stunden.

Tabelle 6

Umsetzungsprodukte aus Talgalkohol und Glykolsäure

| Beispiel | Edukte / Mol | | Produkteigenschaften | | | |
|---|---|---|---|---|---|---|
| | Talg-alkohol | Glykol-säure | Konsistenz, Farbe | Hydroxylzahl theor. | gef. | Säurezahl |
| 26 | 1 | 1 | fest, hellbeige | 176,4 | 181,2 | 4,8 |
| 27 | 1 | 2 | fest, hellbeige | 143,2 | 148,1 | 17,2 |
| 28 | 1 | 4 | fest, hellbeige | 114,1 | 101,2 | 24 |
| 29 | 1 | 8 | fest, weiß | - | - | 37 |

2. b) Monoalkohol x Milchsäure

Herstellvorschrift:

Die in der Tabelle 7 angegebenen Mengen Milchsäure und Talgalkohol wurden in einer Laborrührapparatur mit Destillationsbrücke unter Stickstoff innerhalb 6 - 8 Stunden langsam auf 210 - 220 ° C aufgeheizt. Dabei destillierten das Reaktionswasser und das Wasser aus der Milchsüure (90 %ig in $H_2O$) über.

Um die Reaktion zu vervollständigen, wurde anschließend auf ca. 150 °C abgekühlt und bei dieser Temperatur 30 Minuten lang Wasserstrahlvakuum angelegt. Zur Reinigung des Produktes kühlte man auf ca. 90 °C und versetzte mit Bleicherde Tonsil LFF 80 (5 % bezogen auf Milchsäure), ließ 30 Minuten rühren und saugte dann den Ansatz über eine Heißdampfnutsche mit Filterhilfsmittel ab. Es wurde heiß abgefüllt. Als Katalysator wurde Fascat 2001 (0,5 % bezogen auf Milchsäure) eingesetzt.

Tabelle 7

Umsetzungsprodukte aus Talgalkohol und Milchsäure

| Beispiel | Edukte / Mol | | Reaktions-wasser | Hydroxylzahl | | Säure-zahl | Konsistenz und Farbe |
|---|---|---|---|---|---|---|---|
| | Talg-alkohol | Milch-säure | | theor. | gef. | | |
| 30 | 1 | 1 | 100 % | 167 | 169,1 | 1,9 | wachsartig-weich, weiß |
| 31 | 1 | 2 | 100 % | 137,5 | 142,5 | 11,7 | wachsartig-weich, weiß |
| 32 | 1 | 4 | 100 % | 101,6 | 116,7 | 28,7 | wachsartig-weich, weiß |

17

2. c) Monoalkohol x Milchsäureethylester

Herstellungsvorschrift:

In einer wie unter 2 a) beschriebenen Apparatur wurden Talgfettalkohol und Milchsäureethylester vorgelegt, viermal evakuiert, jeweils mit Stickstoff belüftet und anschließend unter schwachem $N_2$-Strom bis auf 160 °C Badtemperatur aufgeheizt. Sobald bei ca. 60 °C Sumpftemperatur das Reaktionsgemisch aufgeschmolzen war, wurden pro Mol Milchsäureethylester 340 mg einer 30 gew.-%igen Lösung von $NaOCH_3$ in Methanol hinzugegeben. Danach heizte man langsam weiter auf, bis bei ca. 190 °C Badtemperatur (ca. 150 °C Sumpftemperatur) erstmals Ethanol überdestillierte. Die Badtemperatur wurde allmählich bis auf 230 °C erhöht. Als kein Ethanol mehr überdestillierte, ließ man das Reaktionsgemisch bis kurz oberhalb der Kristallisationstemperatur abkühlen und evakuierte dann durch Wasserstrahlvakuum. Man heize wieder langsam bis 150 °C auf und destillierte so das restliche Ethanol im Wasserstrahlvakuum ab. Das Produkt wurde noch heiß abgefüllt.

Umsetzungsprodukte aus Talgalkohol und Milchsäureethylester

Tabelle 7 a

| Beispiel | Edukte / Mol | | Produkteigenschaften | | | | |
|---|---|---|---|---|---|---|---|
| | Talg-alkohol | Milchsäure-ethylester | Konsistenz/Farbe | Ausbeute abdest. Ethanol % | Hydroxylzahl theor. | gef. | Säure-zahl |
| 33 | 1 | 1 | fest, hellgelb | 98 | 168,9 | 176,9 | 0,3 |
| 34 | 1 | 2 | fest, hellgelb | 100 | 138,9 | 151,3 | 0,4 |
| 35 | 1 | 4 | flüssig mit Kristallbrei, schmierig braun | 100 | 102,3 | 145,6 | 1,5 |
| 36 | 1 | 8 | dünnflüssig, schwarz | 96 | 67,1 | - | - |

2. d) Monoalkohol x Lactid

Herstellvorschrift:

In einer wie unter 1. a) beschriebenen Apparatur wurden Alkohol, Lactid und pro Mol Lactid 2,3 ml einer Lösung von 2,5 mg SnCl$_2$ pro ml Ether zusammengegeben. Die Apparatur wurde viermal evakuiert und mit Stickstoff belüftet. Unter schwachem Stickstoffstrom wurden die Komponenten innerhalb von 1 Stunde aufgeheizt bis auf 190 bis 195 °C. Anschließend ließ man noch 3 bis 3,5 Stunden bei 190 bis 195 °C reagieren und füllte dann noch heiß ab.

**Tabelle 8**

Umsetzungsprodukte aus Monoalkoholen und Lactid

| Beispiel | Edukte | | Produkteigenschaften | | |
|---|---|---|---|---|---|
| | Alkohol (je 1 Mol) | Lactid (Mol) | Konsistenz, Farbe | Hydroxylzahl theor. | gef. |
| 37 | Decanol | 0,5 | flüssig, farblos | 244 | 239 |
| 38 | Decanol | 2 | flüssig, farblos | 126 | 117 |
| 39 | Talgfett-alkohol | 0,5 | wachsartig, weiß | 167 | 169 |
| 40 | Talgfett-alkohol | 1 | wachsartig, weiß | 137 | 143 |
| 41 | Talgfett-alkohol | 2 | wachsartig, weiß | 112 | 107 |
| 42 | Talgfett-alkohol | 4 | wachsartig, weiß | 67 | 73 |
| 43 | Behenyl-alkohol | 0,5 | fest, farblos, trübe | 142 | 132 |
| 44 | Behenyl-alkohol | 2 | fest, farblos, trübe | 92 | 88 |

3. Umsetzungsprodukte von Dicarbonsäuren mit Hydroxycarbonsäuren

Herstellvorschrift;
In einem Dreihalskolben mit Rührer und Destillationsbrücke werden Dicarbonsäure und Hydroxycarbonsäure vorgelegt. Unter Stickstoff wird schnell auf 150 °C und dann im Verlauf von 6 Stunden von 150 auf 200 °C hochgeheizt. Dabei spaltet sich bereits der größte Teil des Reaktionswassers, der den Umsatz der Esterkondensation anzeigt, ab. Man läßt den Ansatz auf etwa 150 °C abkühlen, evakuiert vorsichtig auf 10 Torr und vervollständigt den Umsatz bei 200 °C und 10 Torr. Das Produkt wird heiß unter Stickstoff abgefüllt. Die Zusammensetzung der Ansätze und die Oligomereigenschaften sind der Tabelle 9 zu

entnehmen.

**Tabelle 9**

**Umsetzungsprodukte aus Adipinsäure und Glykolsäure**

| Beispiel | Edukte | | Ausbeute | Beschaffenheit |
|---|---|---|---|---|
| | Glycolsäure | Adipinsäure | Reaktionswasser | |
| | Mol | Mol | % | |
| 45 | 1 | 1 | 99 | wachsartig, fest |
| 46 | 1,5 | 1 | 90 | pastenartig, weich |
| 47 | 2 | 1 | 99 | pastenartig, weich |
| 48 | 2,5 | 1 | 95 | wachsartig, weich |
| 49 | 3 | 1 | 98 | wachsartig, weich |
| 50 | 4 | 1 | 96 | wachsartig, weich |
| 51 | 5 | 1 | 93 | wachsartig, hart |
| 52 | 5,5 | 1 | 91 | wachsartig, hart |
| 53 | 6 | 1 | 92 | wachsartig, hart |
| 54 | 20 | 1 | 92 | wachsartig, sehr hart |

4. Umsetzungsprodukte von Glycerin mit Hydroxycarbonsäuren und Fettsäuren

Herstellvorschrift:
Die in den Tabellen 10 - 12 angegebenen Mengen der Edukte wurden in einer Laborrührapparatur mit Destillationsbrücke unter Stickstoff innerhalb 5 Stunden langsam auf 210 - 220 °C aufgeheizt. Als Katalysator wurde Fascat 2001 (0,5 % bezogen auf Milchsäure) eingesetzt. Dabei destillierte sowohl das Reaktionswasser als auch das Wasser aus der eingesetzten Milchsäure (90 %ig in $H_2O$) über.

Um die Reaktion zu vervollständigen, wurde im Anschluß auf ca. 150 °C abgekühlt und bei dieser Temperatur 30 Minuten lang Wasserstrahlvakuum angelegt.

Zur Aufarbeitung des Produktes kühlte man auf ca. 120 °C ab und versetzte mit Bleicherde Tonsil LFF 80 (5 % bezogen auf Milchsäure), ließ 30 Minuten rühren und saugte dann den Ansatz über eine Heißdampfnutsche mit Filterhilfsmittel ab. Das Produkt wurde heiß abgefüllt.

Einzelheiten der Laboransätze und Produkteigenschaften sind in den Tabellen 10 - 12 zusammengefaßt.

## Tabelle 10

Umsetzungsprodukte aus Glycerin, Glykolsäure, Fettsäure

| Bei-spiel | Umsetzungsprodukt aus: | Verh.in Mol | Reaktions-wasser % | Säure-zahl | Hydroxyl-zahl | Aussehen | löslich in |
|---|---|---|---|---|---|---|---|
| 55 | Glycerin/Glykolsäure/ Behensäure | 1:3:3 | 97,8 | 38 | 27 | gelblich,sehr hart,spröde | - |
| 56 | " | 1:12:3 | 91,3 | 54 | 71 | bräunlich-beige, sehr hart,spröde | - |
| 57 | Glycerin/Glykolsäure/ Palmitinsäure | 1:3:3 | 94,4 | 46 | 25 | gelblich,hart, spröde | Aceton |
| 58 | " | 1:12:3 | 90,4 | 79 | - | bräunlich-beige, etwas weicher, aber noch spröde | - |
| 59 | Glycerin/Glykolsäure/ Ölsäure | 1:3:3 | 91,0 | 43 | 35 | dunkelbraun,trüb leicht viskos | n-Hexan |
| 60 | " | 1:12:3 | 89,6 | 62 | 5 | mittelbraun, pastenartig | - |
| 61 | Glycerin/Glykolsäure/ Linolsäure | 1:3:3 | 87,5 | 41 | 31 | hellbraun,klar, leicht viskos | n-Hexan |
| 62 | " | 1:12:3 | 87,8 | 67 | - | hellbraune Paste | - |

## Tabelle 11

Umsetzungsprodukte aus Glycerin, Milchsäure, Fettsäure

| Bei-spiel | Umsetzungsprodukt aus: | Verh.in Mol | Reaktions-wasser % | Säure-zahl | Hydroxyl-zahl | Aussehen | löslich in |
|---|---|---|---|---|---|---|---|
| 63 | Glycerin/Milchsäure/ Behensäure | 1:3:3 | 95,8 | 38 | 31 | gelblich,hart extrem spröde | - |
| 64 | Glycerin/Milchsäure/ Behensäure | 1:12:3 | 100 | 55 | 32 | hellbraun,hart, spröde | Ether |
| 65 | Glycerin/Milchsäure/ Palmitinsäure | 1:3:3 | 100 | 42 | 32 | beige,hart, spröde | Ether |
| 66 | " | 1:12:3 | 100 | 55 | 31 | bräunlich-gelb, viskos,mit star-ker Kristallbil-dung | n-Hexan |
| 67 | Glycerin/Milchsäure/ Ölsäure | 1:3:3 | 99,3 | 42 | 34 | hellbraun,klar, leicht viskos | Ethanol |
| 68 | " | 1:12:3 | 100 | 59 | 27 | mittelbraun, klar, viskos | Ethanol |
| 69 | Glycerin/Milchsäure/ Linolsäure | 1:3:3 | 95,9 | 45 | 40 | gelb, klar, leicht viskos | Ethanol |
| 70 | " | 1:12:3 | 100 | 67 | 50 | hellbraun, klar, viskos | Ethanol |

EP 0 250 994 B1

## Tabelle 12

Umsetzungsprodukte aus Glycerin, Glykolsäure, Milchsäure, Fettsäure

| Bei-spiel | Umsetzungsprodukt aus: | Verh. in Mol | Reaktions-wasser % | Säure-zahl | Hydroxyl-zahl | Aussehen | löslich in |
|---|---|---|---|---|---|---|---|
| 71 | Glycerin/Glykolsäure/ Milchsäure/Behensäure | 1:6:6:3 | 96,9 | 58 | 15 | bräunlich,hart, spröde | - |
| 72 | Glycerin/Glykolsäure/ Milchsäure/Palmitin-säure | 1:6:6:3 | 100 | 66 | 17 | bräunlich-gelb, hart | n-Hexan |
| 73 | Glycerin/Glykolsäure/ Milchsäure/Ölsäure | 1:6:6:3 | 100 | 66 | - | dunkelbraun, klar, hochviskos | Ethanol |
| 74 | Glycerin/Glykolsäure/ Milchsäure/Linolsäure | 1:6:6:3 | 100 | 65 | - | mittelbraun, schwach trüb, viskos | Ethanol |

5. Umsetzungsprodukte von Homo- und Difettsäureglyceriden mit Lactid

Herstellvorschrift:

Die in den Tabellen 13 und 14 angegebenen Mengen Lactid und Fettsäureglycerid wurden in einer üblichen Laborapparatur unter Stickstoff und Rühren innerhalb 1 Stunde auf 195 °C erwärmt.

Man ließ 4 Stunden bei 195 °C reagieren und füllte heiß ab.

Als Katalysator war eine Sn-II-Chlorid-Lösung in Ether verwendet (2,3 ml einer 0,25 %igen Lösung von SnCl$_2$-Ether pro Mol Lactid).

Einzelheiten der Laboransätze und Produkteigenschaften sind in den Tabellen 13 und 14 zusammengefaßt.

Tabelle 13

Umsetzungsprodukte von Glycerinmonoestern und Lactid

| Beispiel | Umsetzungsprodukt aus Lactid und: | Mole Lactid/ Öl-Equivalent | Aussehen und Konsistenz |
|---|---|---|---|
| 75 | Glycerinmonostearat | 0,5 : 1 | weiß und fest |
| 76 | " | 1 : 1 | weiß und fest |
| 77 | " | 2 : 1 | weiß und fest |
| 78 | Glycerinmonooleat | 0,5 : 1 | mittelbraun, klar, leicht viskos |
| 79 | " | 1 : 1 | mittelbraun, klar, viskos |
| 80 | " | 2 : 1 | mittelbraun, klar, hochviskos |

## Tabelle 14

**Umsetzungsprodukte von Glycerinmohoestern und Lactid**

| Beispiel | Umsetzungsprodukt aus Lactid und: | Mole Lactid/ OH-Equivalent | Aussehen und Konsistenz |
|---|---|---|---|
| 81 | Glycerindioleat | 0,5 : 1 | hellbraun, klar, dünnviskos |
| 82 | " | 1 : 1 | hellbraun, klar, dünnviskos |
| 83 | " | 2 : 1 | hellbraun, klar, leicht viskos |

EP 0 250 994 B1

## Tabelle 15

Umsetzungsprodukte von Glycerindiestern und Lactid

| Beispiel | Umsetzungsprodukt aus Lactid und: | Mole Lactid/ OH-Equivalent | Aussehen und Konsistenz |
|---|---|---|---|
| 84 | Glycerindioleat | 4 : 1 | mittelbraun, klar, viskos |
| 85 | " | 8 : 1 | hellbraun, stark trüb, fest |
| 86 | " | 16 : 1 | hellbraun, stark trüb, fest |

EP 0 250 994 B1

Umsetzungsprodukte von Glycerinmonoestern und Lactid

**Tabelle 16**

| Beispiel | Umsetzungsprodukt aus Lactid und: | Mole Lactid/Öl-Equivalent | Aussehen und Konsistenz |
|---|---|---|---|
| 87 | Glycerinmonooleat | 4 : 1 | mittelbraun, klar, hochviskos |
| 88 | " | 8 : 1 | hellbraun, klar, hochviskos |
| 89 | " | 16 : 1 | gelblich, klar, hochviskos bis fest |

Die nachfolgende Tabelle 17 zeigt die Eignung von erfindungsgemäßen Oligohydroxycarbonsäurederivaten (OHCS) zur Konfektionierung mit praxis- und handelsüblichen Desinfektionsmitteln für die unterschiedlichsten Anwendungen insbesondere auf dem Gebiet der Haut-, Schleimhaut- und/oder Wunddesinfektion. Als geeignete Wirkstoffe sind hier beispielsweise zu nennen Halogene und halogenhaltige Präparate, z.B. Jodpräparate, organische Schwermetallverbindungen, quartäre Ammoniumverbindungen, Guanidinderivate, Aldehyde, oxidierende Verbindungen, azide Verbindungen und dergleichen. Die ausgewählten flüssigen OHCS lassen sich zum überwiegenden Teil schon ohne Lösungsmittel mit diesen antimikrobiellen Wirkstoffen mischen. In seltenen Fällen wird die Mischbarkeit erst unter Zuhilfenahme von üblichen Lösungsmitteln, insbesondere Alkoholen erreicht.

Zur Demonstration der Möglichkeiten wurde für einige Beispiele eine Auswahl an desinfizierenden Wirkstoffen getroffen:

Die Mischbarkeit der binären Systeme (99 Gewichtsprozent OHCS, 1 Gewichtsprozent antimikrobieller Wirkstoff) wurde jeweils im Reagenzglas bestimmt.

Beurteilung:

+ :   mischbar ohne Sedimentbildung

- :   nicht mischbar.

Zur Bestimmung der ternären Mischbarkeit wurden OHCS (89 Gewichtsprozent), Lösungsmittel (10 Gewichtsprozent) und antimikrobieller Wirkstoff (1 Gewichtsprozent) wie oben gemischt und beurteilt.

Saccharin - vorgeschlagen als Sekretabsorber - ist mit 5 Gewichtsprozent in allen flüssigen OHCS bis auf das Material des Beispiels 62 löslich.

EP 0 250 994 B1

## Tabelle 17

Mischbarkeit flüssiger OHCS mit antimikrobiellen Wirkstoffen, gegebenenfalls
unter Zuhilfenahme von Lösungsvermittlern

| OHCS | antimikrobielle Wirkstoffe | | | | | | |
|---|---|---|---|---|---|---|---|
| | Chlorhexi-dinlsg. (20 %ig) | Dodigen 1611 (QAV 50 %ig) | Glyoxal (40 %ig) | $H_2O_2$ (35 %ig) | Irgasan DP 300 (100 %ig, Pulver) | Milchsäure (90 %ig) | Carbamid (100 %ig, Pulver) |
| gem. bsp. 2 | - | + | + | - | + | + | + |
| geeignete Lösungsmittel | - | Ethanol Isopropanol n-Propanol Propylen-glykol Dowanol Glycerin | Ethanol Isopropanol n-Propanol Propylen-glykol Dowanol Glycerin | - | Ethanol Isopropanol n-Propanol Propylen-glykol Dowanol Glycerin | Ethanol Isopropa-nol n-Propanol Propylen-glykol Dowanol Glycerin | Ethanol Isopro-panol n-Propanol Propylen-glykol Dowanol Glycerin |
| gem. Bsp. 8 | + | + | + | + | + | + | + |
| geeignete Lösungsmittel | Ethanol, Isopropanol, n-Propanol, Propylenglykol, Dowanol, Glycerin | | | | | | |

**Fortsetzung Tabelle 17**

Mischbarkeit flüssiger OHCS mit antimikrobiellen Wirkstoffen, gegebenenfalls

unter Zuhilfenahme von Lösungsvermittlern

| OHCS | antimikrobielle Wirkstoffe | | | | | | |
|---|---|---|---|---|---|---|---|
| | Chlorhexidinlsg. (20 %ig) | Dodigen 1611 (QAV 50 %ig) | Glyoxal (40 %ig) | $H_2O_2$ (35 %ig) | Irgasan DP 300 (100 %ig, Pulver) | Milchsäure (90 %ig) | Carbamid (100 %ig, Pulver) |
| gem. Bsp. 14 | + | + | + | + | + | + | + |
| geeignete Lösungsmittel | Ethanol, Isopropanol, n-Propanol, Propylenglykol, Dowanol, Glycerin | | | | | | |
| gem. Bsp. 69 | - | + | - | - | + | - | + |
| geeignete Lösungsmittel | - | Ethanol Isopropanol n-Propanol Dowanol | n-Propanol Dowanol | Dowanol | Ethanol Isopropanol n-Propanol | - | Ethanol Isopropanol n-Propanol Dowanol |

In den nachfolgenden Beispielen 90 bis 100 sind Wirkstoffzusammensetzungen für Desinfektionsmittel verschiedenartigster Anwendungsgebiete unter Verwendung der erfindungsgemäßen OHCS angegeben.

Händedesinfektionsmittel

|  | Beispiel 90 |
|---|---|
| Ethanol (MEK vergällt) | 46,0 |
| Isopropanol | 27,0 |
| Benzylalkohol | 1,0 |
| Wasserstoffperoxid | 0,1 |
| C 8/12-Fettsäureglycerid | 0,5 |
| Parfüm | 0,2 |
| OHCS gemäß Beispiel 8 | 25,0 |
| Wasser | Rest |

Hautdesinfektionsmittel

|  | Beispiel 91 | Beispiel 92 |
|---|---|---|
| Isopropanol | 50,0 | 40,0 |
| Chlorhexidindigluconat | 0,5 | 0,5 |
| Wasserstoffperoxid | 0,45 | 0,45 |
| C 8/12-Fettsäureglycerid | 0,5 | 0,5 |
| Saccharin | - | 5,0 |
| Parfüm | 0,1 | 0,1 |
| OHCS gemäß Beispiel 8 | 25,4 | 30,4 |
| Wasser | Rest | Rest |

Schleimhautdesinfektionsmittel

|  | Beispiel 93 | Beispiel 94 |
|---|---|---|
| Ethanol (MEK verg.) | 20,0 | 20,0 |
| Irgasan DP 300 | - | 0,3 |
| Chlorhexidindigluconat | 0,3 | - |
| Wasserstoffperoxid | 0,5 | 0,5 |
| Milchsäure | 0,2 | - |
| Rizinusöl + 40 EO (hydriert) | 0,1 | - |
| Propylenglykol | - | 3,0 |
| OHCS gem. Beispiel 14 | 6,0 | - |
| OHCS gem. Beispiel 8 | - | 10,0 |
| Wasser | Rest | Rest |

Wunddesinfektionsmittel, flüssig

|  | Beispiel 95 | Beispiel 96 | Beispiel 97 |
|---|---|---|---|
| Ethanol (MEK verg.) | 20,0 | 20,0 | - |
| Chlorhexidindigluconat | 0,3 | 0,3 | - |
| Irgasan DP 300 | - | - | 3,0 |
| Wasserstoffperoxid | 0,5 | 0,5 | - |
| Milchsäure | 0,2 | 0,2 | 10,0 |
| Rizinusöl + 40 EO(hydriert) | 0,1 | - | - |
| Phenylsalicylate | 0,1 | - | - |
| Glycerin | - | - | 5,0 |
| Vitamin E | - | - | 0,5 |
| Melissa oficinalis | 1,0 | - | - |
| OHCS gem. Beispiel 14 | 4,0 | - | - |
| OHCS gem. Beispiel 8 | - | 85,0 | - |
| OHCS gem. Beispiel 8 | - | - | 20,0 |
| Wasser | Rest | Rest | Rest |

## Wunddesinfektionsmittel, pulverförmig

| | Beispiel 98 | Beispiel 99 | Beispiel 100 |
|---|---|---|---|
| Percarbamid | 2,0 | 3,0 | - |
| Milchsäure | 15,0 | - | 15,0 |
| Saccharin | 5,0 | - | - |
| Oxoferin | - | 0,2 | - |
| Irgasan DP 300 | - | - | 2,0 |
| OHCS gem. Beispiel 58 | 78,0 | - | - |
| OHCS gem. Beispiel 65 | - | 96,8 | - |
| OHCS gem. Beispiel 54 | - | - | 83,0 |

Verweilzeit-Bestimmung

Die nachfolgend in Tabelle 18 aufgezählten 16 oligomeren Hydroxycarbonsäurederivate der Erfindung wurden in einem in-vitro-Test untersucht. Die Testsubstanzen wurden dabei auf die excidierte Rückenhaut haarloser Ratten aufgetragen und die so behandelte Haut über einen Zeitraum von insgesamt 24 Stunden auf eine Diffusionskammer nach SCHÄFER/STÜTTGEN gelegt, wobei die Dermis mit physiologischer Kochsalzlösung (Kammerwasser) umspült wurde. Die Diffusionskammer wurde auf 35 °C temperiert. In allen Versuchen wurden ca. 60 mg der Prüfsubstanz auf eine kreisrunde Applikationsfläche von 1,77 cm$^2$ - entsprechend der Fläche der Kammeröffnung - aufgetragen. Pastenförmige Hydroxycarbonsäuren wurden zur Viskositätsminderung zuvor auf dem Wasserbad auf ca. 60 °C erwärmt. Die Prüfsubstanz wurde mit einem Mikrospatel auf der Hautfläche verrieben und das Hautstück auf die Kammeröffnung der auf 35 °C erwärmten Glaskammer gelegt. Die Cutis-Seite der Haut befand sich während der Versuchsdauer (24 Stunden) in Kontakt mit der Kammerflüssigkeit (physiologische Kochsalzlösung), die mittels Magnetrührer ständig bewegt wurde.

Die Hautstücke wurden im Verlauf von 8 Stunden stündlich von 2 Mitarbeitern optisch nach folgenden Kriterien hinsichtlich der aufgetragenen Testsubstanzen begutachtet:

Verlust an Glanz / Austrocknung

Farbänderung

Farbintensitätsänderung

Ausbreitung über die behandelte Fläche hinaus.

Zur Nachkontrolle wurden die Hautstücke 24 Stunden auf der Kammeröffnung belassen.

Die nachfolgende Tabelle 18 faßt die Untersuchungen und die dabei erhaltene optische Beurteilung zusammen.

## Tabelle 18

| Substanz Beispiel | Viskosität | Farbe | Applikations-menge $(mg/cm^2)$ | optische Beurteilung |
|---|---|---|---|---|
| 4 | mittel | weiß | 59,5 | keine Ausbreitung, kein Glanzverlust |
| 5 | mittel | weiß | 28,5 | keine Ausbreitung, kein Glanzverlust |
| 18 | mittel | weiß | 30,7 | keine Ausbreitung, kein Glanzverlust |
| 23 | mittel | weiß | 34,7 | keine Ausbreitung, kein Glanzverlust |
| 25 | Paste | weiß | 27,6 | keine Ausbreitung, kein Glanzverlust |
| 32 | mittel | weiß | 29,2 | keine Ausbreitung, kein Glanzverlust |
| 30 | Paste | weiß | 30,3 | nach 1-7 h abnehmender Glanz, nach 24 h matt |
| 67 | niedrig | gelb | 37,9 | nach ca. 3 h Ausbreitung |
| 68 | mittel | gelb | 32,2 | keine Ausbreitung, kein Glanzverlust |
| 74 | mittel | gelb | 32,3 | nach ca. 3 h leichte Ausbreitung |
| 70 | niedrig | gelb | 40,1 | keine Ausbreitung, kein Glanzverlust |
| 80 | hoch | gelb | 40,7 | keine Ausbreitung, kein Glanzverlust |
| 83 | niedrig | gelb | 28,7 | leichte Ausbreitung nach 2 h |
| 89 | Paste | gelb | 29,8 | leichtes Austrocknen nach 6 h |
| 86 | Paste | gelb | 38,0 | keine Ausbreitung, kein Glanzverlust |
| 77 | Paste | weiß | 36,0 | keine Ausbreitung, kein Glanzverlust |

**Patentansprüche**

1. Verwendung von fließfähigen bis festen oligomeren Estern der Milchsäure und/oder der Glykolsäure und ihren dermatologisch verträglichen Derivaten als resorbierbarer Träger und/oder Filmbildner zur Herstellung eines kosmetischen oder pharmazeutischen Mittels zur Abdeckung unverletzter und/oder verletzter Bereiche menschlicher oder tierischer Haut.

2. Verwendung nach Anspruch 1 dadurch gekennzeichnet, daß Oligomere mit einem mittleren Oligomerisationsgrad bis zu etwa 100, vorzugsweise bis zu etwa 50 und insbesondere bis etwa 35 verwendet werden.

3. Verwendung nach Ansprüchen 1 und 2 dadurch gekennzeichnet, daß oligomere Milchsäure- und/oder Glykolsäureester verwendet werden, die über Estergruppen mit anderen über Hydroxyl- und/oder Carboxylgruppen reaktiven Komponenten verknüpft sind.

4. Verwendung nach Ansprüchen 1 bis 3 dadurch gekennzeichnet, daß die oligomeren Milchsäure- und/oder Glykolsäureester mit ein- und/oder mehrfunktionellen Alkoholen und/oder ein- und/oder mehrfunktionellen Carbonsäuren verknüpft sind, die im Fall der mehrfunktionellen Alkohole bevorzugt bis zu vier Hydroxylgruppen aufweisen.

5. Verwendung nach Ansprüchen 1 bis 4 dadurch gekennzeichnet, daß oligomere Umsetzungsprodukte von Milchsäure und/oder Glykolsäure bzw. ihren reaktionsfähigen Derivaten mit monofunktionellen Alkoholen bis zu 22 C-Atomen, insbesondere bis zu 18 C-Atomen verwendet werden.

6. Verwendung nach Ansprüchen 1 bis 4 dadurch gekennzeichnet, daß oligomere Umsetzungsprodukte von Milchsäure und/oder Glykolsäure bzw. ihren reaktiven Derivaten mit Di- und/oder Triolen und/oder deren reaktionsfähigen Derivaten, insbesondere deren Teil- und/oder Vollestern mit Carbonsäuren verwendet werden.

7. Verwendung nach Anspruch 6 dadurch gekennzeichnet, daß oligomere Milchsäure und/oder Glykolsäure enthaltende Glycerinester verwendet werden, die auch körperverträgliche Monocarbonsäuren enthalten können.

8. Verwendung nach Ansprüchen 6 und 7 dadurch gekennzeichnet, daß oligomere Milchsäure- und/oder Glykolsäure-Reste enthaltende Umsetzungsprodukte von Mono-, Di- und/oder Trifettsäureglyceriden mit Milchsäure und/oder Glykolsäure bzw. ihren reaktiven Derivaten verwendet werden.

9. Verwendung nach Ansprüchen 6 bis 8 dadurch gekennzeichnet, daß mit oligomeren Milchsäure- und/oder Glykolsäure-Resten modifizierte natürliche und/oder synthetische Fette, Wachse und/oder Öle verwendet werden.

10. Verwendung nach Ansprüchen 1 bis 9 dadurch gekennzeichnet, daß ölige bis viskos-pastös verstreichbare oligomere Umsetzungsprodukte verwendet werden.

11. Verwendung nach Ansprüchen 1 bis 9 dadurch gekennzeichnet, daß wachsartig bis hart-feste oligomere Umsetzungsprodukte verwendet werden, die insbesondere auch als Pulver oder in Form flüssigemulgierter Massen - bevorzugt als Puder oder in wäßriger Aufbereitung - eingesetzt werden können.

12. Verwendung nach Ansprüchen 1 bis 11 dadurch gekennzeichnet, daß oligomere Träger und/oder Filmbildner mit einem Gehalt an freien Caboxylgruppen verwendet werden.

13. Verwendung nach Ansprüchen 1 bis 12 dadurch gekennzeichnet, daß Träger bzw. Filmbbilner der angegebenen Art eingesetzt werden, deren Permanenz (Verweilzeit auf der Haut als feststellbarer Film) durch den Oligomerisierungsgrad der Umsetzungsprodukte geregelt ist.

14. Verwendung nach Ansprüchen 1 bis 13 dadurch gekennzeichnet, daß die oligomeren Träger und/oder Filmbildner in Abmischung mit Wirkstoffen, z.B. in Abmischung mit hautpflegenden, regenerierenden, desinfizierenden, die Haut-Neubildung anregenden bzw. anderen Wundversorgungsmitteln und/oder zusammen mit pharmakologisch wirksamen Wirkstoffen verwendet werden.

**Claims**

1.  The use of fluid to solid oligomeric esters of lactic acid and/or glycolic acid and dermatologically safe derivatives thereof as resorbable carriers and/or film formers for the production of a cosmetic or pharmaceutical preparation for covering undamaged and/or damaged areas of human or animal skin.

2.  The use claimed in claim 1, characterized in that oligomers having an average degree of oligomerization of up to about 100, preferably of up to about 50 and more preferably of up to about 35 are used.

3.  The use claimed in claims 1 and 2, characterized in that oligomeric lactic acid and/or glycolic acid esters attached by ester groups to other components reactive through hydroxyl and/or carboxyl groups are used.

4.  The use claimed in claims 1 to 3, characterized in that the oligomeric lactic acid and/or glycolic acid esters are attached to monofunctional and/or polyfunctional alcohols and/or monofunctional and/or polyfunctional carboxylic acids which, in the case of the polyfunctional alcohols, preferably contain up to 4 hydroxyl groups.

5.  The use claimed in claims 1 to 4, characterized in that oligomeric reaction products of lactic acid and/or glycolic acid or reactive derivatives thereof with monofunctional alcohols containing up to 22 carbon atoms and more especially up to 18 carbon atoms are used.

6.  The use claimed in claims 1 to 4, characterized in that oligomeric reaction products of lactic acid and/or glycolic acid or reactive derivatives thereof with diols and/or triols and/or reactive derivatives thereof, particularly partial and/or full esters thereof with carboxylic acids are used.

7.  The use claimed in claim 6, characterized in that oligomeric glycerol esters which contain lactic acid and/or glycolic acid and which may also contain monocarboxylic acids compatible with the body are used.

8.  The use claimed in claims 6 and 7, characterized in that oligomeric reaction products containing lactic acid and/or glycolic acid residues of fatty acid mono-, di- and/or triglycerides with lactic acid and/or glycolic acid or reactive derivatives thereof are used.

9.  The use claimed in claims 6 to 8, characterized in that natural and/or synthetic fats, waxes and/or oils modified with oligomeric lactic acid and/or glycolic acid residues are used.

10. The use claimed in claims 1 to 9, characterized in that oily to viscous-pasty, spreadable oligomeric reaction products are used.

11. The use claimed in claims 1 to 9, characterized in that wax-like to hard - solid, oligomeric reaction products, which may even be formulated in particular as powders or in the form of liquid emulsions, preferably in the form of powders or in the form of aqueous preparations, are used.

12. The use claimed in claims 1 to 11, characterized in that oligomeric carriers and/or film formers containing free carboxyl groups are used.

13. The use claimed in claims 1 to 12, characterized in that carriers or film formers of the type mentioned, of which the permanence (residence time on the skin as a fixable film) is regulated through the degree of oligomerization of the reaction products, are used.

14. The use claimed in claims 1 to 13, characterized in that the oligomeric carriers and/or film formers are used in admixture with active substances, for example in admixture with skin-care, regenerating, disinfecting, skin-renewal-promoting or other wound-care agents, and/or together with pharmacologically active agents.

**Revendications**

1.  Utilisation d'esters oligomères liquides à solides de l'acide lactique et/ou de l'acide glycolique et de leurs dérivés dermatologiquement compatibles comme supports résorbables et/ou agents filmogènes pour la préparation d'un produit cosmétique ou pharmaceutique visant à recouvrir des régions intactes et/ou blessées de la peau humaine ou animale.

2. Utilisation selon la revendication 1 caractérisée en ce que les oligomères sont utilisés avec un degré d'oligomérisation moyen jusqu'à environ 100, de préférence jusqu'à environ 50 et, tout particulièrement jusqu'à environ 35.

3. Utilisation selon les revendications 1 et 2 caractérisée en ce qu'elle utilise des esters oligomères d'acide lactique et/ou d'acide glycolique qui sont liés par des radicaux esters avec d'autres composants réactifs par des radicaux hydroxyle et/ou carboxyle.

4. Utilisation selon les revendications 1 à 3 caractérisée en ce que les esters oligomères d'acide lactique et/ou glycolique sont liés avec des alcools monofonctionnels et/ou polyfonctionnels et/ou des acides carboxyliques monofonctionnels et/ou polyfonctionnels qui présentent dans le cas des alcools polyfonctionnels de préférence jusqu'à 4 radicaux hydroxyle.

5. Utilisation selon les revendications 1 à 4 caractérisée en ce qu'elle utilise des produits d'addition oligomères d'acide lactique et/ou d'acide glycolique ou leurs dérivés réactifs avec des alcools monofonctionnels contenant jusqu'à 22 atomes de carbone, en particulier jusqu'à 18 atomes de carbone.

6. Utilisation selon les revendications 1 à 4 caractérisée en ce qu'elle utilise des produits d'addition oligomères d'acide lactique et/ou d'acide glycolique ou leurs dérivés réactifs avec des diols et/ou des triols ou leurs dérivés réactifs, en particulier leurs esters partiels et/ou esters complets avec des acides carboxyliques.

7. Utilisation selon la revendication 6 caractérisée en ce que des esters oligomères de glycérine contenant de l'acide lactique et/ou de l'acide glycolique qui peuvent également contenir des acides monocarboxyliques physiocompatibles sont utilisés.

8. Utilisation selon les revendications 6 et 7 caractérisée en ce que des produits d'addition oligomères contenant des radicaux d'acide lactique et/ou d'acide glycolique de glycérides de monoacides gras, de diacides gras et/ou triacides gras et/ou d'acide glycolique ou de leurs dérivés réactifs sont utilisés.

9. Utilisation selon les revendications 6 à 8 caractérisée en ce que des graisses, cires et/ou huiles naturelles et/ou synthétiques modifiées par des radicaux oligomères d'acide lactique et/ou d'acide glycolique sont utilisées.

10. Utilisation selon les revendications 1 à 9 caractérisée en ce que dos produits d'addition oligomères huileux à pâteux et visqueux qui peuvent être appliqués par râclage sont utilisés.

11. Utilisation selon les revendications 1 à 9 caractérisée en ce que des produits d'addition oligomères cireux à solides et durs qui peuvent être utilisés particulièrement sous forme de poudre ou de masse émulsionnée liquide - en particulier sous forme de poudre ou de préparation aqueuse.

12. Utilisation selon les revendications 1 à 11 caractérisée en ce que des supports oligomères et/ou des agents filmogènes contenant des radicaux carboxyle libres sont utilisés,

13. Utilisation selon les revendications 1 à 12 caractérisée en ce qu'elle met en oeuvre des supports ou agents filmogènes du type susmentionné dont la permanence (durée de séjour sur la peau comme pellicule pouvant être observée) est réglée par le degré d'oligomérisation des produits d'addition.

14. Utilisation selon les revendications 1 à 13 caractérisée en ce que les supports oligomères et/ou agents filmogènes sont utilisés en mélange avec des substances actives, par exemple en mélange avec des substances de soin de la peau, des substances régénérantes, désinfectantes, stimulant la cicatrisation ou d'autres produits de traitement des plaies et/ou avec des substances actives à action pharmacologique.